# EUROPEAN PATENT SPECIFICATION

(11) **EP 1 919 487 B1**
(45) Date of publication and mention of the grant of the patent: **27.06.2012**
(21) Application number: 06849422.8
(22) Date of filing: 27.07.2006
(51) Int. Cl.: A61K 9/00, A61K 31/734, A61K 9/16, A61K 9/50, A61K 9/20

(54) **PARTICULATE COMPOSITIONS COMPRISING ALGINATE AND/OR ALGINIC ACID**
TEILCHENFÖRMIGE ZUSAMMENSETZUNGEN MIT ALGINAT UND/ODER ALGINSÄURE
COMPOSITIONS PARTICULAIRES COMPORTANT DE L'ALGINATE ET/OU DE L'ACIDE ALGINIQUE

(30) Priority: 28.07.2005 GB 0515492
(43) Date of publication of application: 14.05.2008
(73) Proprietor: Reckitt Benckiser Healthcare (UK) Limited, Slough, Berkshire SL1 3UH (GB)
(72) Inventor: JOLLIFFE, Ian, Gordon, Hull HU8 7DS (GB); TRAFFORD, Charles, Hull HU8 7DS (GB); GASEROD, Olav, N-3002 Drammen (NO); Myrvold, Rolf, N- 1389 Heggedal (NO)
(74) Representative: O'Brien, Niall James
(86) International application number: PCT/GB2006/002807
(87) International publication number: WO 2007/113454

(56) References cited:
- EP-A- 0 455 475
- WO-A-03/068246
- GB-A- 1 524 740
- US-A- 4 172 120
- US-B1- 6 258 381
- US-B1- 6 261 601
- BURGER, WACHTER: "Hunnius - Pharmazeutische Wörterbuch", 1998, DE GRUYTER * page 336 *

## Description

The present invention relates to pharmaceutical compositions, and in particular to compositions for the treatment of reflux oesophagitis, gastritis, dyspepsia or septic ulceration or for use as sustained releasing or targeted delivery compositions, as well as to related articles and methods.

Reflux oesophagitis occurs when small amounts of gastric juice, food and/or bile acids pass into the lower part of the oesophagus and cause oesophageal inflammation accompanied by pain which may manifest itself in the form of heartburn.

One approach to the problem of reflux oesophagitis has been to administer a preparation which on contact with gastric acid generates a carbonated gelatinous foam or raft which floats on the stomach contents. When reflux occurs it is this raft which precedes the stomach contents into the oesophagus, thus protecting the mucosa from further irritation. Known preparations of this type include liquid preparations comprising sodium alginate, sodium or potassium bicarbonate and calcium carbonate: Such compositions are sold under the trade marks GAVISCON and GAVISCON ADVANCE and are described in GB-A-1,524,740 and WO 95/11668.

Other such preparations are those in solid form, for example in the form of powders or tablets, such as those which again are sold under the trade mark GAVISCON. Such preparations comprise alginic acid, sodium bicarbonate and calcium carbonate. The alginic acid and the bicarbonate and carbonate react in the aqueous environment of the mouth to form an alginate foam, which is then swallowed. In the acidic stomach environment the alginate is converted back into insoluble alginic acid, which then forms the raft on top of the stomach contents.

It has been found that solid compositions which foam in this manner in the mouth are difficult, and sometimes unpleasant, to swallow. However corresponding compositions in which the alginic acid is replaced by an alginate have their own drawbacks. In general such compositions have extremely poor mouth feel. The alginate is sticky and may cause the composition to stick to the palate, and especially to the teeth.

We did have a degree of success in producing alginate compositions with an improvement in mouth feel and stickiness, and this is described in our earlier patent application WO 03/068246. In this specification there is described tablets comprising an alginate, a bicarbonate and/or carbonate, and a C₂-C₅ polyol or poly(C₂-C₅ alkylene glycol) having a molecular weight of at least 6000, all these components being added together, for blending. However there is a need for an alternative, preferably improved, composition.

In the present invention it is an important object of preferred embodiments to achieve a flowable particulate composition which can be administered directly into the' mouth. The tablets of WO 03/068246 are of course administered directly into the mouth but the issues surrounding the oral administration of tablets are quite different to the issues surrounding the oral administration of particulate compositions, for example tablets may simply be swallowed or may be chewed. Chewing stimulates the release of saliva, which reduces stickiness/gumminess, or the perception thereof. When a particulate composition is administered it potentially has a rapid drying effect in the mouth, and there is no chewing to mitigate that effect.

.The compositions of WO 03/068246 may be prepared by simply mixing the ingredients, and pressing them into tablets. Preferably, however, the ingredients are mixed together and then granulated or agglomerated. In our research work we tested the powder or granulate precursors of the tablets of WO 03/068246 for their suitability for oral administration, but they were found to be unsuitable. They were, at the same time, gritty in the mouth and powdery/dusty. Also they were sticky in the mouth for a considerable time, and it took some effort, on the part of the patient, to clear the mouth.

In accordance with a first aspect of the present invention there is provided an ingestible particulate composition in accordance with claim 1.

Preferably the composition is a flowable particulate, by which we mean that it may be poured from a container, e.g. a sachet, in the manner of sugar or salt.

The function of the agglomerant is to hold the components together, in a form which flows, and yet which substantially does not release into the air fine particulates, i.e. "dust", which could cause a patient to cough or choke.

A particulate composition of the present invention may effervesce in the mouth of the patient; the bicarbonate and carbonate, and the organic acid, preferably form an effervescent couple. This effervescence appears to manifest itself by moderate "fizzing", rather than gross foaming, achieved by the alginic acid tablets mentioned above.

The composition of the present invention preferably comprises an alginate or alginic acid. Most preferably, however, it is an alginate, with no alginic acid present.

When an alginate is present any alginate may be used, but it is especially desirable to use an alkali metal salt of an alginate, such as sodium or potassium alginate. Preferably a low viscosity grade of the alginate is used. These are generally grades of alginate for which the viscosity of a 10% weight/volume aqueous solution, when determined on a Brookfield RVT viscometer using spindle number 3 at 20 r.p.m. at 20 °C, falls within the range of 200 to 1,500 mPa.s. An example of a suitable commercial grade of low viscosity sodium alginate is Protanal LFR 5/60, obtainable from FMC BioPolymer. High viscosity grades of alginate may also be used. These are generally grades of alginate for which the viscosity of a 1% weight/volume aqueous solution, when determined on a Brookfield RVT viscometer using spindle number 3 at 20 r.p.m. at 20 °C, is above 500 mPa.s. An example of a suitable commercial grade of high viscosity sodium alginate is Protanal SF200, also obtainable from FMC BioPolymer. Medium viscosity grades of alginate may also be used, having viscosity above the range defined above for low viscosity grades, but below the range defined above for high viscosity grades.

The compositions of the present invention preferably have a content of alginate and/or alginic acid of 2 to 90 wt%, preferably 10 to 80 wt%, preferably 25 to 75wt%, most preferably 30 to 70wt%; this being the cumulative amount when there is more than one such compound; and being based on the total weight of the components a. b..c. and d.

The compositions of the present invention preferably have a content of alginate and/or alginic acid of 2 to 80 wt%, preferably 6 to 70 wt%, preferably 20 to 60wt%, most preferably 25 to 50wt%; this being the cumulative amount when there is more than one such compound; and being based on the total weight of the composition.

The compositions of the present invention also comprise a bicarbonate and carbonate. Examples of bicarbonates are alkali metal bicarbonates such as sodium and potassium bicarbonate and alkaline earth metal bicarbonates. One or two or more different bicarbonates may be used. Examples of carbonates are alkali metal carbonates such as sodium and potassium carbonate and alkaline earth metal carbonates such as calcium and magnesium carbonate. Further examples are aluminium carbonate and mixed alkali metal carbonates such as sodium glycine carbonate. One or two or more different carbonates may be used. Furthermore one or more bicarbonates may be used with one or more carbonates. Especially preferred combinations are sodium and/or potassium bicarbonate and calcium carbonate.

The carbonate and bicarbonate are present in amounts such that they provide an adequate volume of gas (carbon dioxide) to float the gel or "raft" produced when the alginate and/or alginic acid contacts the gastric acid in the stomach. The rigidity and thickness of the raft will depend, for example, upon the relative amounts of carbonate and/or bicarbonate and on the grade of the alginate and/or alginic acid.

The bicarbonate is suitably present in the compositions of the present invention in an amount of 1 to 60 wt%, preferably 2 to 50 wt%, preferably 5 to 40%, and most preferably 10 to 35wt%; this being the cumulative amount when there is more than one bicarbonate present; and being based on total weight of the components a. b. c. and d.

The bicarbonate is suitably present in the compositions of the present invention in an amount of 1 to 50 wt%, preferably 1.5 to 40 wt%, preferably 4 to 30%, and most preferably 8 to 25wt%; this being the cumulative amount when there is more than one bicarbonate present; and being based on total weight of the composition.

The carbonate is suitably present in the compositions of the present invention in an amount of 1 to 60 wt%, preferably 2 to 50 wt%, preferably 10 to 40 wt%, and most preferably 5 to 30 wt%, and most preferably 10 to 25wt%; this being the cumulative amount when there is more than one carbonate present; and being based on total weight of the components a. b. c. and d.

The carbonate is suitably present in the compositions of the present invention in an amount of 1 to 50 wt%, preferably 1.5 to 40 wt%, preferably 4 to 30%, and most preferably 6 to 20wt%; this being the cumulative amount when there is more than one bicarbonate present; and being based on total weight of the composition.

Both bicarbonate and carbonate are present, preferably in a cumulative amount of 2 to 70 wt%, preferably 10 to 60 wt%, and preferably 20 to 50 wt%, based on total weight of the components a. b. c. and d.

Both bicarbonate and carbonate are present, preferably in a cumulative amount of 1 to 60 wt%, preferably 5 to 50 wt%, and preferably 15 to 40 wt%, based on total weight of the composition.

Approximately equal amounts of the bicarbonate and carbonate may be present in the composition. Alternatively, the composition may comprise more bicarbonate than carbonate. The weight ratio of bicarbonate to carbonate in the composition may suitably be from 1:1 to 3:1, preferably from 1:1 to 2:1.

Preferably the organic acid is a carboxylic acid. Most preferably it is a polycarboxylic acid. Preferably it has 2-5 carboxylic acid groups, more preferably 3-4 carboxylic acid groups, especially 3. Examples of preferred organic acids include citric acid, tartaric acid, malic acid, succinic acid, ascorbic acid, adipic acid and fumaric acid.

The molar ratio of organic acid(s): bicarbonate and/or carbonate (combined weight when both are present) is preferably:
1 (acid): at least 1 (bicarbonate/carbonate); more preferably 1: greater than 1;
more preferably 1: at least 1.5;
more preferably 1: at least 2;
more preferably 1: at least 4;
more preferably 1: at least 5;
and most preferably 1: at least 6.

Preferably the particulate composition contains at least 0.5 wt% organic acid, more preferably at least 2 wt%, more preferably at least 5 wt%, and most preferably at least 8 wt%. Preferably it contains up to 30 wt% organic acid, more preferably up to 20 wt%, most preferably up to 15 wt%. These values denote the cumulative amount when there is more than one organic acid present; and are based on total weight of the components a. b. c. and d.

Preferably the particulate composition contains at least 0.3 wt% organic acid, more preferably at least 1 wt%, more preferably at least 3 wt%, and most preferably at least 5 wt%. Preferably it contains up to 25 wt% organic acid, more preferably up to 18 wt%, most preferably up to 12 wt%. These values denote the cumulative amount when there is more than one organic acid present; and are based on total weight of the composition.

In this invention liquid forms of agglomerant are preferred; including solids which may be liquefied, e.g. by heat, for incorporation into the composition. Preferably the agglomerant is a liquid for part or all of the temperature range 20 to 60°C, at atmospheric pressure. Most preferably it is a liquid at 20°C, at atmospheric pressure.

Suitably the agglomerant is a polymeric or oligomeric compound; preferably a polymeric or oligomeric compound having a molecular weight up to 4000 Daltons. Preferably the molecular weight of the agglomerant does not exceed 2800, more preferably 2500, more preferably 2000, more preferably 1500, more preferably 1000 Daltons. Most preferably its molecular weight does not exceed 600 Daltons.

Preferred compositions of the invention do not contain a cross-linked polyacrylic acid, or polyvinyl pyrrolidone, or acacia.

Preferably its molecular weight is at least 200, more preferably at least 300 Daltons.

Most preferably its molecular weight is about 400 Daltons.

The molecular weight values stated herein are mean values when the agglomerant comprises a series of related compounds representing a range of chain lengths.

The agglomerant may suitably be a hydrophilic surfactant having an HLB value in the range 8-20, preferably 10-18.

The agglomerant preferably comprises a polyoxyalkylene chain, preferably a polyoxyethylene chain.

One suitable agglomerant may be a block copolymer based on ethylene oxide and propylene oxide. These are available under the trade mark PLURONIC.

Another material suitable as an agglomerant is a polyoxyethylene sorbitan fatty acid ester (polysorbate).

Preferred as an agglomerate herein is a polyhydric alcohol, for example a polyhydric monomeric alcohol or a polyhydric polymeric alcohol.

A preferred agglomerant of the first type is a C₂₋₅ polyol. A preferred agglomerant of the second type is a poly(C₂-C₅ alkylene glycol), most preferably polypropylene glycol, polyethylene/polypropylene glycol or, especially, polyethylene glycol.

Especially preferred as an agglomerant in this invention is PEG 400.

Other suitable agglomerants may include lecithin, oils and C₂-C₅ polyols, for example glycerol and propylene glycol.

The agglomerant is preferably present in the compositions of the present invention in an amount of at least 0.01 wt%, more preferably at least 0.05 wt%, more preferably at least 0.1 wt%, and most preferably at least 0.2 wt%. The agglomerant is preferably present in an amount up to 5 wt%, preferably up to 2 wt%, preferably up to 1 wt%, most preferably up to 0.5 wt%. In each case these definitions denote the cumulative amount when there is more than one agglomerant present; and are based on the total amount of the components a. b. c. and d.

The agglomerant is present in the compositions of the present invention in an amount of at least 0.005 wt%, preferably at least 0.02 wt%, more preferably at least 0.05 wt%, and most preferably at least 0.1 wt%. The agglomerant is present in an amount up to 4 wt%, preferably up to 1 wt%, preferably up to 0.6 wt%, most preferably up to 0.4 wt%. In each case these definitions denote the cumulative amount when there is more than one agglomerant present; and are based on the total weight of the composition.

When the agglomerant is a liquid at ambient temperature, as is preferred, its upper limit is preferably the amount beyond which the particulate composition would no longer flow freely under gravity.

The compositions of the present invention may also comprise further, optional components.

For example, the compositions of the present invention preferably comprise a source of divalent and/or trivalent metal ions. Such ions strengthen the raft formed in the stomach. Suitable metal ions are calcium and aluminium. The ions may be provided as part of the bicarbonate and/or carbonate, but may also comprise other anions if desired. For example, suitable sources of calcium ions are calcium carbonate, lactate, chloride, gluconate, phosphate, hydrogen phosphate, sulfate, tartrate or citrate, and suitable sources of aluminium ions are aluminium carbonate, lactate, glycinate or phosphate, aluminium magnesium carbonate, hydroxide or magaldrate, aluminium sodium carbonate hydroxide or aluminium sodium silicate. If used, the calcium ions are preferably present in an amount of from 8 to 800 parts, and the aluminium ions are preferably present in an amount of from 2 to 500 parts, per 500 parts by weight of alginate. Insoluble salts are preferred.

The compositions of the present invention may also comprise one or more colourings, sweetenings, flavourings, pH adjusting ingredients and fillers. When the compositions of the present invention are intended for use as sustained releasing compositions they will also comprise at least one active ingredient suitable for specific delivery to the stomach, such as a drug. Examples of suitable drugs are analgesics (e.g. acetaminophen, ibuprofen, flurbiprofen, naproxen, diclofenac, ketoprofen, choline salicylate, benzydamine, buprenorphine, hydrocortisone, betamethasone, codeine, aspirin); decongestants (e.g. pseudoephedrine, phenylephrine, oxymetazoline, menthol, xylometazoline); cough suppressants (e.g. dextromethorphan, codeine, pholocodine); expectorants (e.g. guaiphenesin, n-acetylcysteine, carbocysteine, bromhexine, ambroxol); antiseptics.(e.g. triclosan, chloroxylenol, amylmetacresol, hexylresorcinol, dichlorobenzyl alcohol, benzyl alcohol, dequalinium chloride, cetylpyridinium chloride); cardiovascular agents (e.g. glyceryl trinitrate); local anaesthetics (e.g. benzocaine, lignocaine); antacid agents (e.g. magnesium trisilicate, aluminium hydroxide, magaldrate); antiulcer agents and/or proton pump inhibitors (PPIs) (e.g. carbenoxolone, sucralfate, cimetidine, ranitidine, nizatidine, famotidine, omeprazole, lanzoprazole, esomeparazole, rabeprazole, pantoprazole); antihistamines (e.g.. loratidine, terfenadine, diphenhydramine, chlorphenhydramine, triprolidine, acrivastine); antinausea agents (e.g. prochlorperazine, sumatriptan); bowel regulatory agents (e.g. diphenoxylate, loperamide, sennosides); antifungal agents (e.g. clotrimazole); antimicrobial agents and antibiotics (e.g. fusafungine, tyrothricin).

The compositions of the invention do not contain cholestyramine.

Active ingredients could be provided with coatings which protect them from detrimental interaction with other components and/or which give release of the active ingredients at a desired site in the gastro-intestinal tract.

Preferably, of course, all components of the invention are ingestible, and deemed acceptable by regulation authorities.

The particulate compositions could be formed into tablets, for example by compression, or by encapsulation within, for example, cellulosic (e.g. HPMC) or gelatine coatings.

Preferably the compositions do not contain magnesium stearate. More preferably they do not contain any stearates or hydrogenated fats. Preferably they do not contain any press aids, lubricants or mould release agents. Preferably they do not contain any tabletting aids. Preferably they do not contain apatite, including carbonated apatite.

Although the motivation behind the present invention is to produce a particulate composition which may be administered directly into the mouth of a patient, the consolidation of such a particulate composition into a tablet, or their incorporation into a, capsule, is not excluded; indeed, it would be a desirable further feature. Use of a particulate composition of the present invention for the production of tablets, and such tablets themselves, represent further aspects of the present invention.

Thus, preferred compositions of the present invention remain in a.flowable form, permitting them to be dispensed straight into the mouth e.g. by spoon or by pouring. Preferably they are in a powder and/or granule form. Preferably they may be regarded as a mixture of powder and granules. Even if they comprise powder they substantially do not release dust into the air. Thus they are without propensity to cause coughing or choking due to inhalation.

Preferably the mean particle.size of the composition as determined using sieve methods is not greater than 1.0mm, and is preferably not greater than 0.5mm. Preferably it is at least 0.1mm. Preferably the particulate composition used in the present invention has substantially no particles which would not pass through a 1mm standard sieve.

In a further aspect the composition of the first aspect consists essentially of the defined components a. b. c. and d. That is to say, any further components are negligible as regards composition properties. Any further components may be impurities but in any case (whether further components are impurities or deliberate minor additions) no single further component is present in an amount greater than 1 wt%, preferably greater than 0.5 wt%, or 0.1 wt%; and preferably where there is a plurality of such components, their cumulative amount is preferably not greater than 8 wt%, preferably not greater than 5 wt%, more preferably not greater than 3 wt%.

In a further aspect of the present invention there is provided a single-pack dosage form (which may otherwise be called a unit dosage pack) containing a single dose of a composition in accordance with the first or second aspect of the present invention. A single pack dosage form could be an ampoule or may be provided by a well of a blister pack, but is preferably a sachet.

Preferably a single-pack dosage form for use in the present invention contains from 0.5 to 5 grams of composition, more preferably from 1 to 2 grams.

Most preferably the single-pack dosage form is adapted to dispense its contents to a point or small area within the mouth, preferably on the tongue, rather than to a wide area. Thus it is preferably a pack which may also be termed a targeted outlet pack. It may, for example, be a tubular ampoule, but is preferably a stick-form sachet. Stick-form sachets are available for food products e.g. sauces and soluble coffee granules. A stick-form sachet comprises, essentially, a slim envelope or tube, preferably formed of flexible material, and sealed at its ends. One end is removed (e.g. torn off) by the user, who can then dispense its contents through the open end e.g. using a pouring action. Preferably a suitable stick-pack sachet has an aspect ratio of at least 2, more preferably at least 3, and most preferably at least 5 (whereas a conventional sachet may have an aspect ratio of, typically, 1.3). Aspect ratio is defined for the purpose of this specification as the ratio of the length of the sachet to the maximum width in its central region (away from the sealed ends) measured when the stick-pack sachet is loaded with its intended single dose of composition of the invention (i.e. the diameter, when the stick-pack sachet is in a cylindrical form).

Alternatively the composition could be provided in a bulk pack containing a composition of the invention together with dosage metering information or means (for example a .scoop or dosing cup).

In accordance with a further aspect of the present invention there is provided a targeted outlet pack which necessarily deposits the composition onto a small area within the mouth, and containing a single dose of an ingestible particulate composition in accordance with the first aspect of the present invention.

The targeted outlet pack may be further defined in accordance with the preceding paragraphs, and is preferably a stick-pack sachet.

The composition within the targeted outlet pack may be as defined with reference to the first or second or third aspects.

In accordance with a further aspect of the present invention there is provided a composition of the invention as defined herein for use in a method of treatment of the human or animal body by therapy.

A composition of the present invention may thus be used in a method of treatment of the human or animal body by therapy, especially use in the treatment of reflux oesophagitis, gastritis, dyspepsia, peptic ulceration or extra-oesophageal gastric reflux condition or for use as a sustained releasing or targeted delivery composition.

The composition of the present invention may be used in the manufacture of a medicament for the treatment of reflex oesophagitis, gastritis, dyspepsia, peptic ulceration or extra-oesophageal gastric reflux condition or for use as a sustained releasing or targeted delivery composition.

The composition of the present invention may be used in a method of treating reflux oesophagitis, gastritis, dyspepsia, peptic ulceration or extra-oesophageal gastric reflux condition or for sustained releasing or targeting a delivery composition, which comprises orally administering to a subject in need thereof or liable to need an effective amount of the composition.

The composition is generally administered in an amount of from 100 to 5,000, preferably 200 to 2,000 mg alginate salt or alginic acid, per dose.

The composition of the present invention is preferably flowable, substantially without clumping, and substantially without releasing powdery or dusty materials which might induce coughing. Any tendency to become overly sticky in the mouth appears to be reduced by the fact that it is not powdery, and by the fact that the acid and/or the effervescence it causes stimulates the release of saliva, and aids dispersion by agitation, preventing clumping. In addition we offer the provisional view that the production of effervescence in the mouth provides the patient with a somewhat pleasant distraction, which aids the process of administration in a subtle way. In the embodiment of the invention which employs a stick-pack article, or another means for directing the particulate composition onto a particular region of the tongue, there is a further benefit; administering the particulate material to large areas of the mouth surfaces is detrimental in terms of the user's perception of the pleasantness of the experience: a large part.of the mouth may thereby become gummy.

The compositions of the present invention may be prepared by mixing the ingredients. It is especially preferred to mix certain components together in particulate form and then granulate them using a suitable granulating agent such as water, a C₂-C₄ alcohol such as ethanol or isopropanol, or a mixture thereof, before adding the remaining components. Other granulating agents may be used, for example povidone and cellulose derivatives such as HPMC and starch paste. A preferred starch paste uses water as the granulating solvent, and povidone is generally used with an ethanol or isopropanol solvent. C₂-C₅ polyols or grades of polyalkylene glycol may also be used as granulating agents, but this function is distinct from the possible use of grades of C₂-C₅ polyols or grades of polyalkylene glycol as agglomerants. The granulating agent grades suitably have higher molecular weight than the agglomerant grades. Preferably the former are solid. Preferably they have a molecular weight above 6,000 Daltons, preferably above 8,000, most preferably in the range 10,000-30,000 Daltons, most preferably 15,000-25,000 Daltons. We have surprisingly found that when a wet granulation is carried out, the amount of granulating agent can be reduced while retaining a satisfactory mouthfeel. A normal granulation process may need a weight ratio of granulating agent to alginate or alginic acid of up to about 1:1. However, using a wet granulation process enables the weight ratio of granulating agent to alginate to be reduced to less than 0.25:1, especially less than 0.15:1, while retaining satisfactory properties.

Components which are suitably granulated in this way are the alginate and/or alginic acid, and the bicarbonate and/or carbonate.

The agglomerant is preferably added after granulation, and dispersed by mixing. The organic acid may in some embodiments be added at the same time but is preferably added later. Further components may be added at the same time as the agglomerant, or at the same time as the organic acid (if added later) or, most preferably, after the agglomerant has been mixed in but before the organic acid has been added.

In accordance with a further aspect of the present invention there is provided the use of a composition in accordance with the first aspect of the present invention. , for the treatment of a patient by administration of the composition into the mouth of the patient.

In accordance with a further aspect of the present invention there is provided the use of a composition in accordance with the first aspect of the present invention in the manufacture of a particulate composition suitable for deposition into the mouth of a patient.

The present invention is further described in the following Examples.

### EXAMPLES

### EXAMPLE 1

A particulate alginate formulation was made as follows:

Alginate granules were made from sodium alginate grade LFR5/60 from FMC BioPolymer, Norway (500g); sodium bicarbonate (267g) Medium Granular; calcium carbonate (Sturcal L) (160g) and PEG 20,000 (60g), as granulating agent; all in powder form. These compounds were mixed in a granulator bowl, dry, for 5 minutes at 270rpm. Purified water (220g - sufficient to give a good consistency), as granulating fluid, was then pumped in over 2 minutes. The wet mass was simultaneously mixed and chopped into small pieces with a chopper blade. The wet mass was dried at a temperature of 50°C in a fluid bed drier for 35 minutes, to a moisture content of less than 5% w/w. The dried granules were milled using a 1.00mm "Conidur" screen, running at 3000rpm, and mixed to homogenise.

The granules were then placed in a ribbon blender mixer (Kemutec, 3 litre). The mixer was set at 120rpm. PEG 400 (3.4g) was added dropwise from a syringe, as agglomerant. When addition of PEG 400 is complete, the composition was mixed at 300rpm for 20 minutes. The following auxiliary ingredients were then added and mixed for a further 10 minutes at 300rpm.

| | |
|---|---|
| Xylitol (bulk sweetener) | 280g |
| Flavourant (peppermint) | 20g |
| Aspartame (intense sweetener) | 10g |
| Acesulfame K (intense sweetener) | 10g |
| Silicon dioxide (moisture scavenger) | 3g |

### Citric acid, fine anhydrous (130g) was then added and mixed in for 5 minutes.

The resulting particulate composition was packed into stick-pack sachets each containing 1.445g of the particulate composition. The particulate composition was a free-flowing, substantially dust free, granule/powder formulation. In addition to good flow and anti-dusting properties it was found to have good mouthfeel properties and to be easy to ingest without leaving unpalatable sticky residues in the mouth.

### EXAMPLE 2

Alginate/bicarbonate/carbonate granules were formed as described in Example 1. PEG 400 (6g) was then mixed in. "Auxiliary ingredients" as described in Example 1 were not added; they were not needed, for test purposes. As in Example 1 the final addition was of citric acid (232g).

The resulting particulate composition shared all the beneficial properties shown by the Example 1 composition.

### EXAMPLE 3

Example 2 was repeated except that:
6g PEG 400 was replaced by 4g Polysorbate 80;
232g citric acid was replaced by 200g tartaric acid; and 280g xylitol was added.

An excellent result was again achieved in preliminary testing work.

### EXAMPLE 4

Example 2 was repeated except:
sodium bicarbonate (267g) was replaced by potassium bicarbonate (100g);
and calcium carbonate was reduced from 160g to 100g.

Again, excellent results are achieved.

### COMPARATIVE EXAMPLES A-C

Examples 1 to 4 achieved excellent results and further experimental work sought to examine the significant factors. The work may be summarised as follows.

Comparative Example A. This corresponded to Example 2 but without the organic acid and without the PEG 400, or any other agglomerant. This produced, in the mouth, a slimy bolts of poor taste, which some trialists could not swallow, but had to spit out. The composition was dust-forming.

Comparative Example B. This corresponded to Example 2 but without the agglomerant. This produced a product with reasonable flow properties, but which was dust-forming, had too much foaming, and poor taste properties.

Comparative Example C. This corresponded to Example 2 but without the citric acid. This had a poor taste and poor mouth feel. In fact, some trialists spat it out.

### EXAMPLE 5

The following composition was made, as a free-flowing particulate composition, by the method described in Example 1.

| **Ingredient** | **(g)** |
|---|---|
| Sodium Alginate LFR 5/60 | 500 |
| Sodium Bicarbonate | 267 |
| Calcium Carbonate | 160 |
| PEG 20,000 | 60 |
| ------------------------ | |
| PEG 400 | 3.4 |
| ----------------------- | |
| Ranitidine Hydrochloride | 75 |
| Aspartame | 10 |
| Acesulfame K | 10 |
| Xylitol CM 170 | 282 |
| Peppermint flavour | 20 |
| Silicon Dioxide (Syloid A1-1P) | 3 |
| ------------------------ | |
| Citric Acid Anhydrous Fine | 130 |
| **Total** | **1520.4 (g)** |

This could be split into 1,000 individual unit doses each of 1.52g.

Again, the alginate, bicarbonate, carbonate and PEG 20,000 were granulated together. The PEG 400 was added and thoroughly mixed in, followed by the remaining ingredients, except for the citric acid. This was mixed in as the last step.

The resulting composition had all the positive attributes of the Example 1 composition, but in addition contained the active agent ranitidine hydrochloride, an anti-ulcer medicament.

### EXAMPLE 6

A free-flowing particulate composition was made by the method described in Example 1. This was mixed with omeprezole enteric coated granules, for treating gastric dysfunctions, and made as follows:

The following particulate precursor composition was made using these starter materials.

### (1) Omeprazole granules

| | |
|---|---|
| Magnesium omeprazole | 10g |
| Lactose anhydrous | 200g |
| Povidone K30 | 15g |
| Water | process aid removed in process |

### (2) Separating layer

| | |
|---|---|
| Omeprazole granules, from (1) | 225g |
| Hydroxypropyl methyl cellulose | 25g |
| Talc | 20g |
| magnesium stearate | 2g |
| Water | process aid removed in process |

### (3) Enteric coating layer

| | |
|---|---|
| Precoated granules, from (2) | 272g |
| Methacrylic acid copolymer ( 30% suspension) | 544g |
| Triethyl citrate | 54g |
| mono & di - glycerides (NF) | 10g |
| Polysorbate 80 | 1g |
| Water | process aid removed in process |

The method was as follows.

### Stage 1 - making omeprazole granules.

1. Prepare granules by mixing together the dry powders and add water with agitation in a high speed mixer granulator.
2. Dry the granules in fluid bed drier.
3. Screen through a 1000µm sieve.

### Stage 2 - separating layer coating.

1. Prepare a solution of the coating ingredients in water (i.e. all those except the granules from stage 1).
2. Put the granules from stage 1 in a fluid bed drier and fluidise.
3. Spray on the coating ingredients adjusting the air flow and temperature to achieve coating without agglomeration.
4. Continue to dry in fluid bed drier.

### Stage 3 - enteric layer coating.

1. Prepare a solution of the coating ingredients in water (i.e. all those except the granules from stage 2).
2. Put the granules from stage 2 in a fluid bed drier and fluidise.
3. Spray on the coating ingredients adjusting the air flow and temperature to achieve coating without agglomeration.
4. Continue to dry in fluid bed drier.

### Stage 4 - incorporation into final product base.

1. Prepare alginate granules as described in Example 1.
2. Add PEG400 and then other ingredients (except the omeprezole granules) as other examples & mix.
3. Add in omeprazole granules and mix in.
4. Fill into sachets & seal.

The final blended composition is as follows:

| **Ingredient** | |
|---|---|
| Sodium Alginate LFR 5/60 | 500 |
| Sodium Bicarbonate | 267 |
| Calcium Carbonate | 160 |
| PEG 20,000 | 60 |
| PEG 400 | 3.4 |
| Omeprazole enteric granules | 881 |
| Aspartame | 10 |
| Acesulfame K | 10 |
| Xylitol | 282 |
| Citric Acid Anhydrous Fine | 130 |
| PEG 20,000 | 60 |
| PEG 400 | 3.4 |
| Peppermint flavour | 20 |
| Silicon Dioxide | 3 |
| **Total** | **2389.8** |

## Claims

1. An ingestible particulate composition comprising:
a. an alginate and/or alginic acid;
b. a bicarbonate and a carbonate;
c. an organic acid; and
d. 0.005 to 4wt% of an agglomerant being a compound which allows the particulate composition to flow yet which substantially does nub release fine particulates into the air, which is a liquid between 20 und 60°C at atmospheric pressure and is selected from the group consisting of a hydrophilic surfactant having an HLB value in the range 8-20' a compound which comprises a polyoxyalkylene chain, a block copolymer based on ethylene oxide and propylene oxide, a polyoxyethylene sorbitan fatty acid ester, a C₂₋₅polyol, a poly(C₂-C₅ alkylene glycol) or lecithin; and wherein the compositions do not contain cholestyramine wherein components a and b are granulated together and component d is added subsequently.

2. A composition according to claim 1 wherein component a. comprises sodium alginate.

3. A composition according to claim 1 or 2 wherein the bicarbonate comprises sodium or potassium bicarbonate.

4. A composition according to any preceding claim wherein the carbonate comprises calcium carbonate.

5. A composition according to any preceding claim containing 0.5 - 20 wt% of the organic acid.

6. A composition according to any preceding claim wherein the organic acid comprises a polycarboxylic acid.

7. A composition according to any preceding claim wherein the agglomerant is a liquid for part or all of the temperature range 20-60°C, at atmospheric pressure.

8. A composition according to any preceding claim wherein the agglomerant comprises being a polymeric or oligomeric compound having a molecular weight up to 4000.

9. A composition according to any preceding claim wherein the agglomerant comprises a poly (C₂-C₅ alkylene glycol) and/or a compound having a polyoxyalkylene chain.

10. A composition according to any preceding claim wherein the agglomerant is polyethylene glycol (PEG), preferably PEG 400.

11. A composition according to any preceding claim consisting of components a. b. c. and d.

12. An ingestible particulate composition according to any of the preceding Claims wherein the ingestible particulate composition is effervescent in the mouth of a patient but not grossly foaming.

13. A composition according to any preceding claim, for use in a method for treating the human or animal body by therapy; especially for use in treating reflux oesophagitis, gastritis, dyspepsia or peptic ulceration.

14. A composition as claimed in any of claims 1 to 13, formed into a tablet.

15. A single-pack dosage form containing a single dose of a composition in accordance with any of claims 1 to 12, the single-pack dosage form being a targeted outlet pack which necessarily deposits the composition onto a small area within the mouth.

16. A single-pack dosage form according to claim 15, being a stick-form sachet.

17. A bulk pack containing a bulk source of a composition in accordance with any of claims 1 to 12. together with dosage metering means or dosage information.

18. Use of a composition according to any of claims 1 to 12 in the manufacture of a medicament for the treatment of reflux oesophagitis, gastritis, dyspepsia or peptic ulceration or extra-oesophogial gastric reflex conditions or for use as a sustained releasing or targeted delivery composition.

19. Use of a composition as defined in any of claims 1 to 12 in the manufacture of a particulate composition suitable for pouring into the mouth of a patient.

20. A process for preparing a composition as defined in any of claims 1 to 12 which comprises granulating together the alginate and the bicarbonate and/or carbonate, followed by mixing in the agglomerant, followed by mixing in the acid.

21. A targeted outlet pack containing a single dose of an ingestible flowable particulate composition, the targeted outlet pack being adapted to deposit the composition onto a small area within the mouth, the composition comprising:
a composition as defined in any of claims 1 to 12.

## Patentansprüche

1. Einnehmbare teilchenförmige Zusammensetzung, enthaltend:
a. ein Alginat und/oder Algensäure;
b. ein Hydrogencarbonat und ein Carbonat;
c. eine organische Säure und
d. 0,005 bis 4 Gew.-% eines Agglomerisationsmittels, bei dem es sich um eine Verbindung handelt, die es der teilchenförmigen Zusammensetzung ermöglicht, zu fließen, jedoch im Wesentlichen keine feinen Teilchen in die Luft freizusetzen, bei der es sich zwischen 20 und 60°C bei Atmosphärendruck um eine Flüssigkeit handelt, und welche aus der aus einem hydrophilen Tensid mit einem HLB-Wert im Bereich von 8-20, einer eine Polyoxyalkylenkette umfassenden Verbindung, einem Blockcopolymer auf Basis von Ethylenoxid und Propylenoxid, einem Polyoxyethylensorbitanfettsäureester, einem C₂₋₅-Polyol, einem Poly(C₂-C₅-Alkylenglykol) und Lezithin bestehenden Gruppe ausgewählt ist, und wobei die Zusammensetzung kein Cholestyramin enthält, wobei die Komponenten a und b zusammen granuliert und anschließend mit der Komponente d versetzt werden.

2. Zusammensetzung nach Anspruch 1, wobei Komponente a. Natriumalginat umfasst.

3. Zusammensetzung nach Anspruch 1 oder 2, wobei das Hydrogencarbonat Natrium- oder Kaliumhydrogencarbonat umfasst.

4. Zusammensetzung nach einem der vorhergehenden Ansprüche, wobei das Carbonat Calciumcarbonat umfasst.

5. Zusammensetzung nach einem der vorhergehenden Ansprüche mit 0,5-20 Gew.-% an organischer Säure.

6. Zusammensetzung nach einem der vorhergehenden Ansprüche, wobei die organische Säure eine Polycarbonsäure umfasst.

7. Zusammensetzung nach einem der vorhergehenden Ansprüche, wobei es sich bei dem Agglomerisationsmittel in einem Teil oder dem gesamten Temperaturbereich von 20 bis 60°C bei Atmosphärendruck um eine Flüssigkeit handelt.

8. Zusammensetzung nach einem der vorhergehenden Ansprüche, wobei das Agglomerisationsmittel eine polymere oder oligomere Verbindung mit einem Molekulargewicht von bis zu 4000 umfasst.

9. Zusammensetzung nach einem der vorhergehenden Ansprüche, wobei das Agglomerisationsmittel einen Poly(C₂-C₅-alkylenglykol) und/oder eine Verbindung mit einer Polyoxyalkylenkette umfasst.

10. Zusammensetzung nach einem der vorhergehenden Ansprüche, wobei es sich bei dem Agglomerisationsmittel um Polyethylenglykol (PEG), vorzugsweise PEG 400 handelt.

11. Zusammensetzung nach einem der vorhergehenden Ansprüche, bestehend aus den Komponenten a., b., c. und d.

12. Einnehmbare teilchenförmige Zusammensetzung nach einem der vorhergehenden Ansprüche, wobei die einnehmbare teilchenförmige Zusammensetzung im Mund eines Patienten ausbraust, jedoch nicht übermäßig aufschäumend ist.

13. Zusammensetzung nach einem der vorhergehenden Ansprüche zur Verwendung bei einem Verfahren zur Behandlung des menschlichen oder tierischen Körpers durch Therapie, insbesondere zur Verwendung bei der Behandlung von Rückflussösophagitis, Gastritis, Dyspepsie oder Magengeschwüren.

14. Zusammensetzung nach einem der Ansprüche 1 bis 13, als Tablette geformt.

15. Einzelpackungsdosierungsform, enthaltend eine einzelne Dosis einer Zusammensetzung nach einem der Ansprüche 1 bis 12, wobei es sich bei der Einzelpackungsdosierungsform um eine Packung mit zielgerichtetem Auslass handelt, die die Zusammensetzung zwangsmäßig in einer kleinen Region im Mund deponiert.

16. Einzelpackungsdosierungsform nach Anspruch 15, bei der es sich um einen stäbchenförmigen Beutel handelt.

17. Vorratspackung, enthaltend eine Vorratsquelle einer Zusammensetzung nach einem der Ansprüche 1 bis 12 zusammen mit Mitteln zur dosierten Abmessung oder Dosierungsinformationen.

18. Verwendung einer Zusammensetzung nach einem der Ansprüche 1 bis 12 bei der Herstellung eines Medikaments zur Behandlung von Rückflussösophagitis, Gastritis, Dyspepsie oder Magengeschwüren oder extraösophagialen gastrischen Rückflussleiden oder zur Verwendung als Zusammensetzung mit anhaltender Freisetzung oder gezielter Zuführung.

19. Verwendung einer Zusammensetzung nach einem der Ansprüche 1 bis 12 bei der Herstellung einer teilchenförmigen Zusammensetzung, die sich dafür eignet, in den Mund eines Patienten gegossen zu werden.

20. Verfahren zur Herstellung einer Zusammensetzung nach einem der Ansprüche 1 bis 12, bei dem man das Alginat und das Hydrogencarbonat und/oder Carbonat zusammen granuliert und anschließend mit dem Agglomerisationsmittel mischt und anschließend mit der Säure mischt.

21. Packung mit gezieltem Auslass, enthaltend eine einzelne Dosis einer einnehmbaren fließfähigen teilchenförmigen Zusammensetzung, wobei die Packung mit dem gezielten Auslass so ausgelegt ist, dass die Zusammensetzung in einer kleinen Region im Mund deponiert wird, wobei die Zusammensetzung Folgendes umfasst:
eine Zusammensetzung nach einem der Ansprüche 1 bis 12.

## Revendications

1. Composition particulaire ingérable comprenant :
a. un alginate et/ou de l'acide alginique ;
b. un bicarbonate et un carbonate ;
c. un acide organique ; et
d. 0,005 à 4 % en poids d'un agent d'agglomération, qui est un composé qui permet à la composition particulaire de couler et néanmoins de ne libérer pratiquement pas de fines matières particulaires dans l'air, qui est un liquide entre 20 et 60 °C à pression atmosphérique et qui est choisi parmi un tensioactif hydrophile ayant une valeur d'HLB dans la plage de 8-20, un composé qui comprend une chaîne polyoxyalkylène, un copolymère séquencé à base d'oxyde d'éthylène et d'oxyde de propylène, un ester d'acide gras et de sorbitane polyoxyéthylénique, un polyol en C₂₋₅, un poly(alkylèneglycol en C₂-C₅) ou la lécithine ;
la composition ne contenant pas de cholestyramine et les composants a et b étant granulés ensemble et le composant d étant ajouté par la suite.

2. Composition selon la revendication 1 dans laquelle le composant a comprend de l'alginate de sodium.

3. Composition selon la revendication 1 ou 2 dans laquelle le bicarbonate comprend du bicarbonate de sodium ou de potassium.

4. Composition selon l'une quelconque des revendications précédentes dans laquelle le carbonate comprend du carbonate de calcium.

5. Composition selon l'une quelconque des revendications précédentes contenant 0,5-20 % en poids de l'acide organique.

6. Composition selon l'une quelconque des revendications précédentes dans laquelle l'acide organique comprend un acide polycarboxylique.

7. Composition selon l'une quelconque des revendications précédentes dans laquelle l'agent d'agglomération est un liquide sur une partie ou la totalité de la plage de température de 20-60 °C, à pression atmosphérique.

8. Composition selon l'une quelconque des revendications précédentes dans laquelle l'agent d'agglomération comprend un composé polymère ou oligomère ayant une masse moléculaire allant jusqu'à 4 000.

9. Composition selon l'une quelconque des revendications précédentes dans laquelle l'agent d'agglomération comprend un poly(alkylèneglycol en C₂-C₅) et/ou un composé ayant une chaîne polyoxyalkylène.

10. Composition selon l'une quelconque des revendications précédentes dans laquelle l'agent d'agglomération est un polyéthylèneglycol (PEG), de préférence le PEG 400.

11. Composition selon l'une quelconque des revendications précédentes constituée des composants a, b, c et d.

12. Composition particulaire ingérable selon l'une quelconque des revendications précédentes, la composition particulaire ingérable étant effervescente dans la bouche d'un patient mais ne moussant pas excessivement.

13. Composition selon l'une quelconque des revendications précédentes, destinée à être utilisée dans un procédé pour le traitement du corps humain ou animal par thérapie ; en particulier destinée à être utilisée dans le traitement de l'oesophagite peptique, d'une gastrite, de la dyspepsie ou d'un ulcère gastroduodénal.

14. Composition selon l'une quelconque des revendications 1 à 12, mise sous forme d'un comprimé.

15. Forme pharmaceutique en emballage individuel comprenant une dose unique d'une composition selon l'une quelconque des revendications 1 à 12, la forme pharmaceutique en emballage individuel étant un emballage à orifice de sortie ciblé qui dépose nécessairement la composition sur une petite zone à l'intérieur de la bouche.

16. Forme pharmaceutique en emballage individuel selon la revendication 15, qui est un sachet en forme de bâtonnet.

17. Emballage vrac contenant une source en vrac d'une composition selon l'une quelconque des revendications 1 à 12, conjointement avec des moyens de dosage ou des informations de dosage.

18. Utilisation d'une composition selon l'une quelconque des revendications 1 à 12 dans la fabrication d'un médicament pour le traitement de l'oesophagite peptique, d'une gastrite, de la dyspepsie ou d'un ulcère gastroduodénal ou d'affections de reflux gastrique extra-oesophagien ou destinée à être utilisée comme composition à libération prolongée ou à administration ciblée.

19. Utilisation d'une composition selon l'une quelconque des revendications 1 à 12 dans la fabrication d'une composition particulaire appropriée pour être versée dans la bouche d'un patient.

20. Procédé pour la préparation d'une composition selon l'une quelconque des revendications 1 à 12 qui comprend la granulation de l'alginate et du bicarbonate et/ou du carbonate ensemble, suivie de l'ajout et du mélange de l'agent d'agglomération, suivis de l'ajout et du mélange de l'acide.

21. Emballage à orifice de sortie ciblé contenant une dose unique d'une composition particulaire fluie ingérable, l'emballage à orifice de sortie ciblé étant conçu pour déposer la composition sur une petite zone à l'intérieur de la bouche, la composition comprenant une composition selon l'une quelconque des revendications 1 à 12.
